## Europäisches Patentamt

(19) **European Patent Office**

**Office européen des brevets**

(11) Veröffentlichungsnummer: **0 124 657**
**B1**

(12) # EUROPÄISCHE PATENTSCHRIFT

(45) Veröffentlichungstag der Patentschrift:
11.03.87

(51) Int. Cl.⁴: **C 07 D 213/64**

(21) Anmeldenummer: **83201773.5**

(22) Anmeldetag: **14.12.83**

(54) Verfahren zur Herstellung von 3,5,6-Trichlor-1H-pyridin-2-on.

(30) Priorität: **12.03.83 DE 3308800**

(43) Veröffentlichungstag der Anmeldung:
**14.11.84 Patentblatt 84/46**

(45) Bekanntmachung des Hinweises auf die Patenterteilung:
**11.03.87 Patentblatt 87/11**

(84) Benannte Vertragsstaaten:
**CH DE FR GB IT LI**

(56) Entgegenhaltungen:
**CHEM. OF HET. COMP. PYRID. AND ITS DER.,
Supplement Teil 3, Seiten 800-803, John Wiley & Sons,
New York, USA;
J. ORG. CHEM., Band 26, Dezember 1961, Seiten
4949-4955; D.J. COOK et al.: "Bromination studies of
alkyl-substituted 2-pyridones and 2-quinolones"
J. AGR. FOOD CHEM., Band 14, Nr. 3, Mai/Juni 1966,
Seiten 304-306; R.H. RIGTERINK et al.: "Synthesis and
insecticidal activity of some 0,0-dialkyl
0-3,5,6-trihalo-2-pyridyl phosphate and
phosphorothioates"
RECUEIL, Band 70, Seiten 182-190; H.J. DEN HERTOG
et al.: "Reactions of bromo-derivatives of 2- and
3-ethoxypyridine when heated with hydrochloric acid
solution"**

(73) Patentinhaber: **Rütgerswerke Aktiengesellschaft,
Mainzer Landstrasse 217, D-6000 Frankfurt a.Main 1 (DE)**

(72) Erfinder: **Orth, Winfried, Dr., Am Schachtelgraben 28,
D-6733 Hassloch/Pfalz (DE)**
Erfinder: **Fickert, Werner, Dr., Stockacher Strasse 14,
D-6800 Mannheim 61 (DE)**

**Beschreibung**

Die Erfindung betrifft ein neues Verfahren zur Herstellung von 3,5,6-Trichlor-1H-pyridin-2-on. Diese Verbindung ist ein wichtiges Zwischenprodukt bei der Synthese von Insektiziden.

Als Synthesemöglichkeiten für diese Verbindung kennt man die Chlorierung von 6-Brom-2-äthoxypyridin und die anschliessende Spaltung und Umhalogenierung des 6-Brom-3,5-dichlor-2-äthoxypyridins sowie die Chlorierung von 6-Chlor-1H-pyridin-2-on [den Hertog, De Bruyn; Recueil 70 (1951), 182—190]. Beide Chlorierungsreaktionen erfolgen mit wässrigem Wasserstoffperoxid und wässriger Salzsäure. Sie lassen sich, wie beschrieben, lediglich in kleinen Laboransätzen mit höchstens Grammengen erfolgreich durchführen. Bei grösseren Mengen laufen nicht kontrollierbare Nebenreaktionen ab, die die Ausbeute am gewünschten Produkt drastisch senken. Eine wirtschaftliche, halbtechnische Darstellung von 3,4,6-Trichlor-1H-pyridin-2-on ist auf diesen Wegen nicht möglich.

Aus EP-A-00 30 214 ist eine Direktsynthese aus Trichloracetylchlorid und Acetonitril bekannt. Abgesehen davon, dass diese Umsetzung mit hochtoxischen Ausgangsprodukten in einer aufwendigen Druckreaktion erfolgt, entstehen dabei sowohl das gewünschte 3,5,6-Trichlor-1H-pyridin-2-on als auch 2,3,5,6-Tetrachlorpyridin, so dass diese beiden verwandten Stoffe noch mühsam voneinander getrennt werden müssen.

Aufgabe der Erfindung war es daher, ein Verfahren zur Herstellung von 3,5,6-Trichlor-1H-pyridin-2-on zu liefern, bei dem diese Verbindung aus einer gut zugänglichen Ausgangssubstanz in möglichst einfachen Verfahrensschritten in guter Ausbeute und Reinheit auch in grossen, technischen Ansätzen wirtschaftlich hergestellt werden kann.

Gelöst wurde diese Aufgabe durch ein Verfahren, das dadurch gekennzeichnet ist, dass man bei einer Temperatur von 15 bis 30°C Chlorgas auf eine Lösung von 6-Chlor-1H-pyridin-2-on in einer wässerigen Carbonsäurelösung einer Carbonsäure, die bei der Reaktionstemperatur flüssig und gegen Chlorgas inert ist, derart einwirken lässt, dass man das Chlorgas lediglich in den sich über der Lösung befindlichen Raum einleitet.

Die wirtschaftlich einfachste Darstellung von 3,5,6-Trichlor-1H-pyridin-2-on ist die direkte Umsetzung des leicht erhältlichen 6-Chlor-1H-pyridin-2-ons mit Chlor. Leider führte dies bislang nicht zum Erfolg, weshalb wohl auch den Hertog und de Bruyn den Weg über die indirekte Chlorierung mit Wasserstoffperoxid und Chlorwasserstoff beschritten.

Beim Einleiten von Chlorgas in eine Lösung von 6-Chlor-1H-pyridin-2-on erfolgt eine extrem exotherme Reaktion, und als Folge davon laufen unkontrollierte Zersetzungsreaktionen ab. Diese unerwünschten Reaktionen sind auch nicht dadurch zu unterdrücken, dass man die Reaktionspartner nur in mässig konzentrierter Form aufeinander einwirken lässt, dass man z.B. nur in eine verdünnte Lösung von 6-Chlor-1H-pyridin-2-on ein mit einem Trägergas verdünntes Chlor einleitet.

Überraschenderweise aber gelingt die schonende und selektive Chlorierung von 6-Chlor-1H-pyridin-2-on zum 3,5,6-Trichlor-1H-pyridin-2-on, wenn man Chlorgas über eine Lösung von 6-Chlor-1H-pyridin-2-on in einer wässrigen Carbonsäurelösung leitet und die Temperatur im Bereich von 15 bis 30°C hält. Die von der Lösung aus dem Gasraum aufgenommene Chlormenge und -verteilung ermöglicht eine schonende und dennoch wirtschaftliche Durchführung der gewünschten Chlorierungsreaktion.

Ausgangsprodukt für die Umsetzung kann sowohl reines als auch technisches 6-Chlor-1H-pyridin-2-on sein. In einer weiter vereinfachten Ausführungsform des erfindungsgemässen Verfahrens wird direkt das bei der Herstellung des 6-Chlor-1H-pyridin-2-ons aus dem leicht zugänglichen 2-Chlor-6-methoxypyridin anfallende Reaktionsgemisch ohne vorherige Abtrennung des Reaktionsproduktes erhalten. Hierzu wird eine Suspension aus 2-Chlor-6-methoxypyridin auf 100°C erwärmt und portionsweise mit etwa der doppelten molaren Menge konzentrierter Salzsäure versetzt, wobei die Reaktionsmischung unter Rückfluss gehalten wird. Diese Säurezugabe dauert etwa 2—4 Stunden. Danach schliesst sich eine 15- bis 20stündige Nachreaktion bei der Siedetemperatur des Gemisches an. Während dieser Ätherspaltungsreaktion entweicht Methylchlorid, das in reiner Form aufgegangen und für andere Synthesen eingesetzt werden kann. Das verbleibende Reaktionsgemisch dient als Ausgangsprodukt für die Chlorierungsreaktion. Es wird in der Wärme mit einer flüssigen Carbonsäure oder wässrigen Carbonsäurelösung versetzt und auf 15—30°C abgekühlt.

Geht man bei der Chlorierungsreaktion von reinem 6-Chlor-1H-pyridin-2-on aus, so wird dies in einer wässrigen etwa 30- bis 70%igen Carbonsäurelösung gelöst. Die Menge der Carbonsäure soll ausreichend sein, das 6-Chlor-1H-pyridin-2-on bei der Reaktionstemperatur in Lösung zu halten.

Als Carbonsäuren bieten sich solche an, die bei Reaktionstemperatur, d.h. im Bereich von 15—30°C gegen Chlor inert sind und die bei dieser Temperatur flüssig sind, wie etwa Essig-, Propion-, Butter- oder Valeriansäure aber auch solche, die im Alkylrest substituiert oder verzweigt sind, wie z.B. Methoxyessig-, Dimethylessig-, Methyl-äthylessig- oder i-Valeriansäure.

Die Chlorierungsreaktion erfolgt in einem geschlossenen Gefäss, das mit einem Rührer und mindestens zwei verschliessbaren Öffnungen versehen ist, durch die das Gas in den sich über der Lösung befindlichen Gasraum ge- und gegebenenfalls abgeleitet werden kann. Das Reaktionsgefäss sollte zusätzlich eine leicht Probenentnahme ermöglichen. Die Lösung wird unter Kühlung gerührt, und über diese Lösung wird Chlorgas geleitet, wobei der Gasraum zweckmässigerweise unter einem leichten Überdruck von etwa $50 \cdot 10^2 - 200 \cdot 10^2$ Pa steht. Die Chlorzuführung muss so bemessen sein, dass die Reaktionstemperatur den vorgeschriebenen Bereich nicht überschreitet. Sie dauert etwa 5–10 Stunden.

Während der Reaktion fällt das gebildete 3,5,6-Chlor-1H-pyridin-2-on aus, so dass sich eine Suspension bildet.

Nach beendeter Umsetzung wird überschüssiges Chlorgas durch Zugabe einer geringen Menge Natriumsulfit beseitigt. Dadurch ergibt sich eine bessere Farbstabilität des Endprodukts, das nach Neutralisation des Reaktionsgemisches abfiltriert, gewaschen und getrocknet wird.

Beispiel:

In einem 10-l-Dreihalskolben werden zu einer auf 100°C erwärmten und gut gerührten Mischung aus 861 g (6 Mol) 2-Chlor-6-methoxypyridin und 100 ml Wasser Verlauf von 2,5 h 1167 g (12 Mol) konz. Salzsäure, chem. rein, gleichmässig getropft. Anschliessend erfolgt eine 16-stündige Nachreaktionszeit bei Rückflusstemperatur. Danach werden 5000 ml einer 60%igen Essigsäurelösung zugegeben und anschliessend unter leichtem Überdruck (104 Pa) während 7 h 980 g Chlorgas in den Gasraum über der Lösung geleitet, wobei durch Kühlung die Temperatur des Reaktionsgemisches auf 20–2°C gehalten wird. Nach beendeter Chlorzugabe werden 20 g Natriumhydrogensulfit zugegeben. Die Suspension wird noch weitere 15 min gerührt. Daraufhin werden bei 20–30°C 1120 g 50%ige Natronlauge zugetropft, und anschliessend wird das Produkt abgesaugt und so lange mit Wasser gewaschen, bis das Filtrat neutral reagiert. Das bei 100°C getrocknete Endprodukt hat einen Schmelzpunkt von 175°C (Lit. 174–175°C), Ausbeute: 1100 g = 93% der Theorie.

## Patentansprüche

1. Verfahren zur Herstellung von 3,5,6-Trichlor-1H-pyridin-2-on, dadurch gekennzeichnet, dass man bei einer Temperatur von 15 bis 30°C Chlorgas auf eine Lösung von 6-Chlor-1H-pyridin-2-on in einer wässrigen Carbonsäurelösung einer Carbonsäure, die bei der Reaktionstemperatur flüssig und gegen Chlorgas inert ist, derart einwirken lässt, dass man das Chlorgas lediglich in den sich über der Lösung befindlichen Raum einleitet.

2. Verfahren nach Anspruch 1, dadurch gekennzeichnet, dass die Reaktionstemperatur bei 20–25°C gehalten wird.

3. Verfahren nach Anspruch 1 und 2, dadurch gekennzeichnet, dass der Gasraum unter einem leichten Überdruck von $50 \cdot 10^2$–$200 \cdot 10^2$ Pa steht.

4. Verfahren nach Anspruch 1 bis 3, dadurch gekennzeichnet, dass 2-Chlor-6-methoxy-pyridin der Ätherspaltung unterworfen und das dabei entstehende 6-Chlor-1H-pyridin-2-on direkt anschliessend ohne vorherige Abtrennung chloriert wird.

5. Verfahren nach Anspruch 4, dadurch gekennzeichnet, dass nach der Ätherspaltung die Carbonsäure dem noch warmen Reaktionsgemisch zugegeben wird.

## Claims

1. A process for producing 3,5,6-trichloro-1H-pyridine-2-on, characterized in, that chlorine gas is allowed to react at a temperature in the range of 15 to 30°C with a solution of 6-chloro-1H-pyridine-2-on in an aqueous carboxylic acid solution which, at the reaction temperature, is liquid and inert to chlorine gas and chlorinating in that manner that the chlorine gas is passed only into a the gas space located above said solution.

2. The process as set forth in claim 1, further comprising maintaining the temperature between 20° and 25°C.

3. The process as set forth in claims 1 and 2, further comprising maintaining a light overpressure between $50 \cdot 10^2$ and $200 \cdot 10^2$ Pa in the gase space.

4. The process as set forth in claims 1 to 3, further comprising carrying out an ether cleavage reaction of 2-chloro-6-methoxypyridine and chlorinating the so formed 6-chloro-1H-pyridine-2-on directly without prior separation.

5. The process as set forth in claim 4, after said ether cleavage said carboxylic acid is added to said still-hot reaction mixture.

## Revendications

1. Procédé de préparation de 3,5,6-trichloro-1H-pyridine-2-one à partir de 6-chloro-1H-pyridine-2-one, caractérisé par le fait que de la 6-chloro-1H-pyridine-2-one est dissoute dans une solution aqueuse d'acide carboxylique d'un acide carboxylique, liquide à la température de la réaction et inerte à l'égard du chlore gazeux, et chlorée à une température dans la domaine de 15–30°C dans une enceinte réactionnelle par introduction de chlore gazeux dans l'espace gazeux qui se trouve au-dessus de la solution.

2. Procédé selon la revendication 1, caractérisé par le fait que la température réactionnelle est maintenue à 20–25°C.

3. Procédé selon la revendication 1 et 2, caractérisé par le fait que l'espace gazeux se trouve sous une légère surpression de $50 \cdot 10^2$–$200 \cdot 10^2$ Pa.

4. Procédé selon la revendication 1 à 3, caractérisé par le fait que la chloruration à lieu directement après la scission de l'éther à partir de la 2-chloro-méthoxy-pyridine, sans isolement de la 6-chloro-1H-pyridine-2-one.

5. Procédé selon la revendication 4, caractérisé par le fait qu'après scission de l'éther, l'acide carboxylique est ajouté au mélange réactionnel encore chaud.